# EUROPÄISCHE PATENTSCHRIFT

(11) **EP 0 733 371 B1**
(45) Veröffentlichungstag und Bekanntmachung des Hinweises auf die Patenterteilung: **19.06.2002**
(21) Anmeldenummer: 96103663.9
(22) Anmeldetag: 08.03.1996
(51) Int. Cl.: A61K 38/48

(54) **Mittel zur subkutanen Verabreichung von Protein C**
Agent for the subcutaneous application of protein C
Agent pour l'application sous-coutanée de la protéine C

(30) Priorität: 21.03.1995 DE 19510260; 04.03.1996 DE 19608218
(43) Veröffentlichungstag der Anmeldung: 25.09.1996
(73) Patentinhaber: Baxter Aktiengesellschaft, 1221 Wien (AT)
(72) Erfinder: Eibl, Johann, Dr., 1180 Wien (AT); Schwarz, Hans-Peter, 1180 Wien (AT)
(74) Vertreter: Kolb, Helga, Dr. Dipl.-Chem.

(56) Entgegenhaltungen:
- EP-A- 0 471 660
- PEDIATRIC RESEARCH, Bd. 37, Nr. 4, April 1995, Seite 163A XP002006345 LESLEY G. MITCHELL ET AL: "A single subcutaneous (SQ) injection of protein C concentrate can maintain therapeutic levels for up to 77 hours"
- THROMBOSIS AND HAEMOSTASIS , Bd. 63, Nr. 1, 1990, Seiten 48-53, XP000573858 OKAJIMA K. ET AL: "Effect of protein C and activated protein C on coagulation and fibrinolysis in normal human subjects"
- ADVANCES IN APPLIED BIOTECHNOLOGY SERIES, Bd. 11, 1990, Seiten 83-89, XP002006347 SCHWARZ H.P. ET AL: "Monoclonal antibody purified protein C concentrate"
- PRESPECTIVES IN DRUG DISCOVERY AND DESIGN, Bd. 1, 1993, Seiten 503-520, XP002006348 S. BETTY YAN ET AL: "Recombinant human protein C, protein S and thrombomodulin as antithrombotics"
- REYNOLDS J.E.F.: "MARTINDALE The Extra Pharmacopoeia" 1993 , THE PHARMACEUTICAL PRESS , LONDON XP002006349 * Seite 647 - Seite 648 *

## Beschreibung

Die Erfindung betrifft die Verwendung einer Zusammensetzung zur Herstellung eines pharmazeutischen Mittels, das zur subkutanen Injektion von Protein C geeignet ist.

Protein C wird in der Leber synthetisiert und zirkuliert im Blut als ein inaktives, zweikettiges Protein oder Zymogen in einer Konzentration von 4µg/ml. Bei der Aktivierung durch den Thrombin-Thrombomodulin-Komplex an der Oberfläche des Gefässwandendothels wird das Aktivierungspeptid von Protein C am N-terminalen Ende der schweren Kette des Zymogens abgespalten, und es wird aktiviertes Protein C, eine Serinprotease, gebildet.

Protein C kann aus Plasma oder einer Plasmafraktion, wie dem Gerinnungsfaktorkonzentrat, auch Prothrombin-Komplex genannt, gereinigt und gegebenenfalls in vitro aktiviert werden. Das dabei erhaltene Produkt kann durch im Blut befindliche Viren, wie sie häufig im Plasma vorkommen, kontaminiert sein. Aus diesem Grunde führt man während des Produktionsprozesses eine Reihe von Schritten durch, um Viren zu inaktivieren oder zu eliminieren.

In neuerer Zeit hat man Protein C und dessen aktivierte Form, Derivate und Mutanten davon, mit Hilfe rekombinanter DNA-Technologie hergestellt.

Patienten, die an einem Mangel an Protein C leiden, zeigen häufig thromboembolische Komplikationen, wie ein Purpura fulminans-ähnliches Syndrom. Purpura fulminans in neugeborenen, homozygoten Kindern mit Protein C-Mangel konnte mit einem hoch-gereinigten Protein C-Konzentrat erfolgreich behandelt werden. Die Behandlung begann mit einer Dosis von 20 bis 40 Einheiten/kg, viermal täglich. Die Therapie wurde über 8 Monate bei 100 E/kg/Tag fortgesetzt (H.P. Schwarz et al, Blood, Band 76, Nr. 10, 15.Nov. 1990, Erg. 1, S. 520a, Abstr. 2070).

Protein C in seiner aktivierten Form hat einen antikoagulativen Effekt beim proteolytischen Abbau von Gerinnungsfaktor Va, dem Cofaktor für die Faktor Xainduzierte Prothrombin-Aktivierung (Thrombinbildung), und Faktor VIIIa, dem Cofaktor für die Faktor IXa-induzierte Faktor X-Aktivierung.

In der Literatur wird beschrieben, dass aktiviertes Protein C (APC) Endotoxin-induzierte Sepsis und septischen Schock in einem Baboon-Tierversuch verhindert (Taylor et al, J. Clin. Invest., Band 79, März 1987, S. 918-925). Eine weitere Verwendung von aktiviertem Protein C ergibt sich aus seiner antithrombotischen Funktion, die von Gruber et al aufgezeigt wurde (Blood, Band 73, Nr.3, 15. Feb. 1989, S. 639-642). Von diesen Autoren wird vorgeschlagen, dass die APC-Verabreichung unter arteriellen Fliessbedingungen unmittelbar zu antithrombotischen Effekten führen kann.

Aktiviertes Protein C selbst führt auch zur Thrombolyse im Tierversuch mit Ratten (EP-0 519 903). Dabei ist es wichtig, dass die thrombolytische Präparation von aktiviertem Protein C keine Verunreinigungen, die entweder vom Ausgangsmaterial oder vom Produktionsprozess herrühren können, wie virale Kontaminierungen, oder Thrombin und Serumamyloid P enthält.

Überraschenderweise hat sich gezeigt, dass auch Protein C-Zymogen für die Thrombolyse-Behandlung geeignet ist (EP-0519 900). Es wurde festgestellt, dass Protein C-Mangel durch Plasmin, dem aktiven Enzym der Fibrinolyse, herbeigeführt wird. Dieser Mangel zeigt sich dadurch, dass das Koagulations- und Fibrinolyse-System nicht im Gleichgewicht sind, was zur Bildung von Thromben und Reokklusion führt. Protein C behebt diesen Mangelzustand und verbessert somit die Thrombolysetherapie.

Mit Hilfe eines bisher unbekannten Mechanismus ist Protein C-Zymogen auch in der Lage, die Mikrozirkulation zu verbessern und klinische Äquivalente der Shwartzman-Reaktion zu behandeln und zu verhindern (EP-0 514 367).

Ausserdem hat sich durch die Versuche eine noch wesentlich breitere Anwendbarkeit von Protein C ergeben. Unerwarteterweise hat man festgestellt, dass Schmerzreaktionen, Entzündungen und Gefässschäden im Carageenin-induzierten Rattenpfoten-Modell der Hyperanalgesie durch Protein C inhibiert werden (EP-0 533 210).

Protein C oder aktiviertes Protein C wurde bisher an Patienten intravenös, und zwar entweder prophylaktisch oder bei thromboembolische Komplikationen, zur Erzielung eines unmittelbaren Effektes, verabreicht. Okajima et al (Thrombosis and Haemostasis, 1990, Band 63(1) S. 48-33) injizierten intravenös aktiviertes Protein C in einer Dosis von 24µg/kg, nicht-aktiviertes Protein C in einer Dosis von 0,15 mg/kg. Sie stellten fest, dass die Halbwertszeit für APC 23 Minuten und für Protein C 10,9 h beträgt, bestimmt durch die Verlängerung der aktivierten partiellen Thromboplastin-Zeit.

Aus diesem Grunde ist es oft erforderlich, hohe Dosen zu verabreichen oder häufige Verabreichungen von Protein C und/oder aktiviertem Protein C durchzuführen. Obwohl einige Derivate von Protein C oder aktiviertem Protein C eine verlängerte Halbwertszeit in vivo besitzen, bedeutet die Verabreichung von Protein C oder aktiviertem Protein C aufgrund der langen Infusionszeiten immer noch eine Belastung für den Patienten.

EP-A-0 471 660 beschreibt Protein C-haltige pharmazeutische Präparationen und deren Verwendung bei der Behandlung von schmerzhaften Zuständen, die sowohl durch akute oder auch chronische entzündlichen Prozesse auftreten können. Dieser Effekt der antinociceptiven Wirkung der Protein C-haltigen pharmazeutischen Präparation wird sowohl durch intravenöse Gabe als auch durch subkutane Verabreichung im gleichen Masse erzielt.

Die wiederholte Anwendung intravenöser Injektionen ist für den Patienten nicht nur zeitaufwendig, sondern führt auch zu Nebeneffekten. Es können sich bei der intravenösen Injektion eine Reihe von anaphylaktischen Reaktionen ergeben. Vor allem bei Langzeitbehandlungen kann es zu einer Okklusion bzw. Thrombosierung und auch zur Infektion der Vene ider der venösen Zugänge (Katheter, implantierte Katheter, wie PORT-A-CAT®) an der Injektionsstelle kommen. Dadurch können lebensbedrohliche Zustände, wie Embolien, insbesondere Pulmonalembolien, auftreten. Bei Patienten mit kleinen Venen, wie Babies, ist es für den Arzt darüber hinaus schwierig, die Nadel in die Vene einzuführen.

Aufgabe der Erfindung ist es, neue pharmazeutische Mittel zur Verfügung zu stellen, welche die leichte und komplikationslose Verabreichung von Protein C erlauben.

Die vorstehende Aufgabe wird durch pharmazeutische Mittel gelöst, welche für die subkutane Verabreichung von Protein C geeignet sind. Protein C kann dabei als Zymogen oder in seiner aktivierten Form, als natives Protein C, als Derivat oder Mutante vorliegen. Ein natives Protein C ist definitionsgemäss natürlichen Ursprungs und ist beispielsweise in Plasma oder einer Plasmafraktion, wie Prothrombin-Komplex, enthalten. Ein gleichartiges natives Protein C kann jedoch auch aus entsprechenden Zellkulturen hergestellt werden bzw. ein rekombinantes Protein darstellen. Das zur subkutanen Injektion verwendete Mittel umfasst Protein C und einen pharmazeutisch annehmbaren Träger und ist geeignet für die Verabreichung einer wirksamen Dosis an Protein C an Patienten mit Protein C-Mangel.

Es hat sich überraschenderweise herausgestellt, dass sogar das native Protein C für die subkutane Injektion geeignet ist. Voraussetzung dafür ist die unvermutet rasche Wiederfindung (recovery) von therapeutischen Mengen an Protein C im Blut des Patienten nach einer subkutanen Injektion. Trotz einer unverändert kurzen Halbwertszeit des nativen Protein C ist daher die effektive Anhebung der Protein C-Konzentration im Blut möglich.

Dies war vor allem im Hinblick auf den Stand der Technik (US-5,358,932) überraschend, der modifizierte Protein C-Moleküle mit einer verlängerten Halbwertszeit vorschlägt, um das Problem der relativ kurzen Halbwertszeit von nativem Protein C zu vermeiden.

Die bevorzugte tägliche Dosis liegt im Bereich von 5 bis 500 E/kg, am meisten bevorzugt zwischen 10 und 200 E/kg. Die wirksame Menge an Protein C ist abhängig von der Protein C-Konzentration im Blut des Patienten und der Protein C-Konzentration, wie sie zur Prophylaxe oder Therapie erforderlich ist. Die bevorzugte Mindestkonzentration an Protein C in der verabreichungsfertigen Lösung beträgt 200 E/ml, am meisten bevorzugt eine Konzentration im Bereich von 250 bis 1000 E/ml. Im Gegensatz zu den Präparationen des Standes der Technik, die intravenös verabreicht werden, ist das Volumen des erfindungsgemässen Mittels im allgemeinen klein und beträgt ca. 1 bis 10 ml. Es ist bevorzugt, ziemlich konzentrierte Protein C-Lösungen zu injizieren.

Kleine Volumina werden vorzugsweise als verabreichungsfertige Lösungen in Fertigspritzen zur Verfügung gestellt. Die Spritzen zur sc-Verabreichung von Protein C weisen besonders dünne, relativ lange Nadeln auf. Das verabreichungsfertige Flüssigpräparat ist vorzugsweise stabilisiert und bei Kühlschranktemperatur über einen Zeitraum von mehreren Wochen lagerfähig. Als Stabilisatoren werden beispielsweise Aminosäuren, Zucker, aber auch Tenside, vor allem nichtionische Tenside, wie Tween oder Triton verwendet. Auch Polyalkylenglykole, wie z.B. Polyethylenglykol, sind für die Stabilisierung des Protein C geeignete Substanzen.

Ebenso kann aber auch ein flüssig-tiefgefrorenes Protein C-Präparat in einer spritze zur subkutanen Injektion zur Verfügung gestellt werden.

Bei Verwendung von lyophilisierten Protein C-Präparaten muss die Kühlkette nicht unbedingt eingehalten werden. Ein diesbezügliches Set umfasst einen Behälter mit lyophilisiertem Protein C und eine Spritze, die zur subkutanen Injektion von Protein C geeignet ist. Entsprechende Anweisungen sind dann auf einem Beipackzettel zur Auflösung des Präparates in Wasser zur Injektion oder in Puffer bzw. einer stabilisierenden Lösung angegeben.

Grössere Mengen an Protein C-Lösung können beispielsweise mit entsprechenden Pumpen über einen Zeitraum von 10 Min. bis etwa 5 h verabreicht werden.

Protein C kann als Prothrombin-Komplex, der Protein C enthält, oder als ein hoch-gereinigtes Protein C-Konzentrat, welches mindestens 80%, vorzugsweise mindestens 90% (G/G) rein im Verhältnis zum Gesamtprotein ist, verabreicht werden. Vorteilhafterweise enthält das erfindungsgemässe verwendete Mittel zusätzlich Cofaktoren von Protein C, Beispielswiese Protein S und/oder den antikoagulatorisch wirksamen Faktor V.

Das Mittel gemäss der Erfindung enthält Protein C, welches aus einer biologischen Quelle, wie Blut, Plasma oder einer Plasmafraktion isoliert und vorzugsweise zur Inaktivierung eines möglicherweise vorhandenen Virus behandelt ist. Das Mittel kann auch mit Hilfe einer rekombinanten Methode hergestellt sein.

Die Verwendung gemäss der Erfindung eignet sich besonders in Verfahren zur Behandlung von Patienten, wobei es prophylaktisch oder therapeutisch an Patienten verabreicht wird, die unter einer Reihe der vorstehend beschriebenen Erkrankungszuständen leiden. Unter den medizinischen Indikationen sind vererbter und erworbener Protein C-Mangel zu nennen. In jedem Falle wird der Gehalt an Protein C oder aktiviertem Protein C im Blut auf normale und sogar über dem Normalen liegende Konzentrationen (von 70 bis 200, vorzugsweise 100%) eingestellt.
Bei der Verabreichung von aktiviertem Protein C ist die benötigte Dosis etwa 1/10 der für das Zymogen angegebenen Menge. Bei gleichhzeitiger Verwendung von Cofaktoren bzw. Wirkungsverstärkern kann eine entsprechend geringere Dosis an Protein C genügen.

Überraschenderweise konnte im Kaninchenversuch gezeigt werden, dass selbst eine einmalige subkutane (sc) Injektion eines Protein C-Konzentrats mit einer Dosis im Bereich von 100 bis 1000 µg/kg das therapeutische Niveau bis zu 100 h aufrechterhielt. Je nach der verabreichten Protein C-Dosis, kann dieser Zustand durch höhere Protein C-Dosen sogar noch über einen längeren Zeitraum aufrechterhalten werden. So ist es z.B. möglich, an Patienten, die an Protein C-Mangel leiden, zweimal wöchentlich eine subkutane Injektion zu verabreichen und dadurch das erforderliche Niveau an Protein C aufrechtzuerhalten. Dies ist einer intravenösen Injektion vorzuziehen, insbesondere da sich auf diese Weise Patienten zuhause selbst behandeln können.

Es war nicht zu erwarten, dass man Protein C im Blutkreislauf bereits kurze Zeit nach der sc-Injektion vorfindet. Vielmehr wurde angenommen, dass der Hauptteil des Protein C aufgrund der relativ kurzen Halbwertszeit abgebaut würde. Überraschenderweise stellte es sich heraus, dass man therapeutische Mengen an Protein C im Blut von Säugern bereits etwa 30 Min. nach der Verabreichung von Protein C vorfindet.

Der Vorteil der erfindungsgemässen Verabreichung liegt vor allem darin, dass das Mittel über einen langen Zeitraum hin Protein C in den Blutkreislauf einbringt. Durch die sc-Injektion entsteht ein entsprechendes Depot, von dem eine kontinuierliche Abgabe der Substanzen in den Blutkreislauf erfolgt. Damit wird das Problem der kontinuierlichen Verabreichung von Protein C, welche aufgrund der limitierten Halbwertszeit im Blut erforderlich ist, überwunden.

Das sc-injizierte Protein C liegt dann im Blut in nichtmodifizierter Form vor. Es erfolgt keine Aktivierung von Protein C-Zymogen durch die subkutane Verabreichung.

Die folgenden Beispiele sollen die Erfindung näher erläutern.

### Beispiel 1

### Subkutane Verabreichung von Protein C an ein Kaninchen

¹²⁵I-radioaktiv markiertes Protein C wurde subkutan an ein Kaninchen verabreicht (200 µl/bei einer Konzentration von 0,16 µg/ml). In bestimmten Zeitabständen nach der Verabreichung wurden Blutproben entnommen. Plasma aus diesen Blutproben wurde mit einer SDS-PAGE aufgetrennt und das markierte Protein C mit einem Röntgenfilm detektiert. Ein Gehalt an markiertem Protein C in nicht-abgebauter Form wurde schon nach 30 Minuten im Blut gefunden und war über den Beobachtungszeitraum von 180 Minuten konstant hoch.

### Beispiel 2

### Subkutane Verabreichung von Protein c an einen Patienten

Ein Patient mit schwerem angeborenen Protein C-Mangel wurde mit 1500 Einheiten Protein C in einem Volumen von 20 ml über 2 h behandelt. Die Verabreichung erfolgte mittels einer Pumpe subkutan in die Haut der Bauchdecke. In bestimmten Zeitabständen wurde der Gehalt an Protein C im Blut amidolytisch bestimmt. Es wurden die folgenden Werte gefunden:

| Zeit nach Verabreichung (h) | Aktivität (E/ml) |
|---|---|
| 0 | 0 |
| 6 | 0,73 |
| 12 | 0,90 |
| 24 | 0,59 |
| 48 | 0,26 |

## Patentansprüche

1. Verwendung einer Zusammensetzung zur Herstellung eines Arzneimittels für subkutane Injektion, enthaltend Protein C und einen pharmazeutisch annehmbaren Träger zur Aufrechterhaltung eines Protein C-Gehalts im Blut von 70 bis 200 % bezogen auf die normale Konzentration im Blut, gegen Protein C Defizienz.

2. Verwendung nach Anspruch 1, wobei die bevorzugte tägliche Dosis im Bereich von 5-500 E/kg liegt.

3. Verwendung nach Anspruch 1, **dadurch gekennzeichnet, daß** die Zusammensetzung einen Prothrombin-Komplex enthält.

4. Verwendung nach Anspruch 1, wobei das Protein C zumindest 80 % rein ist.

5. Verwendung nach Anspruch 1, wobei das Protein C zumindest 90 % rein ist.

6. Verwendung nach Anspruch 1, wobei das Protein C ein rekombinant hergestelltes Protein C darstellt.

7. Verwendung nach Anspruch 1, wobei das Protein C aus einer biologischen Quelle isoliert ist.

8. Verwendung nach Anspruch 1, wobei das Protein C ein natives Protein C darstellt.

9. Verwendung nach Anspruch 7, **dadurch gekennzeichnet, daß** die biologische Quelle Blut, Plasma oder eine Plasmafraktion darstellt.

10. Verwendung nach Ansprüchen 1 bis 5, 7 bis 9, wobei Protein C zur Inaktivierung von im Blut vorkommenden Viren behandelt worden ist.

11. Verwendung nach Ansprüchen 1 bis 10, wobei Protein C in aktivierter Form vorliegt.

## Claims

1. Use of a composition for producing a medicament for subcutaneous injection, containing protein C and a pharmaceutically acceptable excipient for maintaining a protein C level in the blood of 70 to 200%, based on the normal concentration in the blood, for protein C deficiency.

2. Use according to claim 1, wherein the preferred daily dose lies in the range from 5-500 units/kg.

3. Use according to claim 1, **characterised in that** the composition contains a prothrombin complex.

4. Use according to claim 1, wherein the protein C is at least 80% pure.

5. Use according to claim 1, wherein the protein C is at least 90% pure.

6. Use according to claim 1, wherein the protein C is a protein C produced in recombinant manner.

7. Use according to claim 1, wherein the protein C is isolated from a biological source.

8. Use according to claim 1, wherein the protein C is a native protein C.

9. Use according to claim 7, **characterised in that** the biological source is blood, plasma or a plasma fraction.

10. Use according to claims 1 to 5, 7 to 9, wherein protein C has been treated to inactivate viruses occurring in the blood.

11. Use according to claims 1 to 10, wherein protein C is present in activated form.

## Revendications

1. Utilisation d'une composition pour la fabrication d'un médicament pour l'injection sous-cutanée, contenant de la protéine C et un porteur pharmaceutique admissible, pour maintenir dans le sang un taux de 70 à 200 % de protéine C, par rapport à la concentration normale dans le sang, contre la déficience de protéine C.

2. Utilisation selon la revendication 1, la dose quotidienne préférée se situant dans la fourchette de 5 à 500 u/kg.

3. Utilisation selon la revendication 1, **caractérisée en ce que** la composition contient un complexe de prothrombine.

4. Utilisation selon la revendication 1, la protéine C présentant une pureté d'au moins 80 %.

5. Utilisation selon la revendication 1, la protéine C présentant une pureté d'au moins 90 %.

6. Utilisation selon la revendication 1, la protéine C étant une protéine C fabriquée de façon recombinante.

7. Utilisation selon la revendication 1, la protéine C étant isolée à partir d'une source biologique.

8. Utilisation selon la revendication 1, la protéine C étant une protéine C native.

9. Utilisation selon la revendication 7, **caractérisée en ce que** la source biologique est du sang, du plasma ou une fraction de plasma.

10. Utilisation selon l'une quelconque des revendications 1 à 5, 7 à 9, la protéine C ayant été préalablement traitée pour l'inactivation des virus éventuellement présents dans le sang.

11. Utilisation selon l'une quelconque des revendications 1 à 10, la protéine C étant présente sous forme activée.
